# EUROPÄISCHE PATENTSCHRIFT

(11) **EP 2 131 800 B1**
(45) Veröffentlichungstag und Bekanntmachung des Hinweises auf die Patenterteilung: **19.01.2011**
(21) Anmeldenummer: 08735089.8
(22) Anmeldetag: 08.04.2008
(51) Int. Cl.: A61F 5/058, A61F 13/04

(54) **SYSTEM ZUR HERSTELLUNG EINES ORTHOPÄDISCHEN SPLINTS AUS EINEM CASTMATERIAL MIT WENIGSTENS EINEM ALS FLACHMATERIALBAHN VORLIEGENDEN THERMOPLASTISCHEN CASTMATERIAL**
SYSTEM FOR PRODUCING AN ORTHOPEDIC SPLINT MADE OF A CAST MATERIAL HAVING AT LEAST ONE THERMOPLASTIC CAST MATERIAL PRESENT AS A FLAT MATERIAL WEB
SYSTÈME POUR LA FABRICATION D'UNE CLAVETTE ORTHOPÉDIQUE CONSTITUÉE D'UN MATÉRIAU DE MOULE AYANT AU MOINS EN TANT QUE NAPPE DE MATÉRIAU PLANE LE PRÉSENT MATÉRIAU DE MOULE THERMOPLASTIQUE

(30) Priorität: 12.04.2007 DE 102007017196
(43) Veröffentlichungstag der Anmeldung: 16.12.2009
(73) Patentinhaber: Karl Otto Braun GmbH & Co. KG., 67752 Wolfstein (DE)
(72) Erfinder: LANGEN, Günter, 67752 Wolfstein (DE)
(74) Vertreter: Langöhrig, Angelika Beate
(86) Internationale Anmeldenummer: PCT/EP2008/002770
(87) Internationale Veröffentlichungsnummer: WO 2008/125249

(56) Entgegenhaltungen:
- EP-A- 0 061 642
- WO-A-97/13479
- US-A- 4 454 873
- US-A- 5 520 621
- US-A1- 2004 024 337

## Beschreibung

Die Erfindung betrifft ein System zur Herstellung eines orthopädischen Splints aus einem Castmaterial mit wenigstens einem als Flachmaterialbahn vorliegenden thermoplastischen Castmaterial.

So werden seit langer Zeit neben dem herkömmlichen Gipsverbandmaterial alternative Verbandmaterialien eingesetzt, die auf Kunststoffbasis gebildet sind. Diese besitzen gegenüber Gipsverbänden Vorteile, da sie verbesserte mechanische Eigenschaften besitzen und wasserunempfindlich und folglich abwaschbar sind sowie aufgrund der schnellen Anlegbarkeit und Aushärtung und wegen ihres geringen Gewichts einen erhöhten Tragkomfort und eine verbesserte Mobilität gewährleisten. Darüber hinaus sind sie im Gegensatz zu Gipsmaterialien für Röntgenstrahlen durchlässig und ermöglichen so Röntgennachuntersuchungen ohne dass der Verband entfernt werden muss. Dabei werden sowohl herkömmliche Gipsverbandmaterialien als auch auf Kunststoff basierende Verbandmaterialien im Wesentlichen aus einem organischen oder anorganischen textilen Trägermaterial und einem darauf aufgebrachten Gips- bzw. Kunststoffmaterial gebildet, wobei bei den Kunststoffmaterialien zwischen irreversibel härtbaren und thermoplastischen, reversibel verformbaren Materialien unterschieden werden muss.

Als irreversibel härtbare Kunststoffmaterialien sind in erster Linie wasserhärtbare Polyurethansysteme bekannt, wobei nach Eintauchen in Wasser die Verbandmaterialien innerhalb einer Zeit aushärten, die das fachgerechte Anlegen und Modellieren des Verbandmaterials am Körper eines Menschen oder Tieres erlauben. Solange der Kunststoff nicht ausgehärtet ist, können die einzelnen Lagen des Verbandmaterials miteinander verklebt werden, wodurch letztendlich ein aus mehreren Schichten bestehender Verband erhalten werden kann.

Bei thermoplastischen, reversibel verformbaren Verbandmaterialien, wie sie gemäß der vorliegenden Erfindung eingesetzt werden sollen, wird die Selbstklebeeigenschaft durch Erwärmen des thermoplastischen Kunststoffs auf die jeweilige Erweichungstemperatur oder darüber erzielt. Beim Abkühlen erstarrt das Material wieder, wobei es auch bei Temperaturen unterhalb des Schmelzpunktes für einige Zeit plastisch modellierbar bleibt. Nach dem Erstarren des thermoplastischen Harzes wird ein mehrlagig miteinander verbundenes Verbandsystem erhalten.

Dabei weisen die irreversibel härtbaren Materialien auf Polyurethanbasis gegenüber den thermoplastischen gewisse Nachteile auf, da aufgrund der Eigenschaft der Wasserhärtbarkeit eine feuchtigkeitsfreie Produktion und eine aufwendige Verpackung des Produktes, die die Verpackung für Wasser und Luft undurchlässig macht, erforderlich ist. Darüber hinaus sind entsprechende Harzrezepturen verhältnismäßig kostspielig und bei Einhaltung möglichst feuchtigkeitsfreier Bedingungen ist die Lagerstabilität vergleichsweise gering. Ferner können Polyurethanharze aufgrund der enthaltenen Isocyanate die Haut reizen und gesundheitsschädliche Reaktionen hervorrufen.

Ein entsprechender orthopädischer Splint sowie ein Castmaterial sind beispielsweise aus der WO 02/054983 bekannt. Bei dem hier eingesetzten Castmaterial handelt es sich um ein wasserhärtendes System, das als härtbare Kunststoffkomponente zumeist reaktive Polyurethanpräpolymere enthält, die durch Kontakt mit Wasser irreversibel aushärten. Die DE 199 07 043 B4 dagegen schützt ein thermoplastisches Verbandmaterial, welches aus einem mit thermoplastischem Kunststoff beschichteten Textilsubstrat besteht und vorzugsweise in Rollenform konfektioniert ist. Es kann in einem sogenannten Ausgabe- oder Dispenserbehälter vorliegen und als quasi Endlosband in Form einer Flachmaterialbahn entnehmbar sein.

Des Weiteren ist ein Castmaterial zur Herstellung eines orthopädischen Splints aus der US 4,454,873 A bekannt, wobei das Castmaterial ein als Flachmaterialbahn vorliegendes thermoplastisches Castmaterial ist, das zur Erzielung einer Verformbarkeit aktivierbar ist.

Ein alternatives Castmaterial auf Basis thermoplastischer Materialien beschreibt die US 2004/024337 A1, die ein zweischichtiges Castmaterial offenbart mit einer Trägerschicht aus einem flexiblen thermoplastischen Material und eine Pufferschicht aus einem elastischen thermoplastischen Material.

Für Splintanwendungen ergeben sich nun folgende Probleme bei thermoplastischen Casten. Grundsätzlich müssen thermoplastische Castmaterialien für Splintanwendungen eine Mindestdicke aufweisen, um genügend Steifigkeit und Stabilität sicherzustellen. Diese Mindestdicke beträgt ca. 3 bis 4 mm. Zwar kann eine entsprechende Mindestdicke auch in einem einlagigen Splintaufbau erreicht werden, analog zu üblichen Splintkonstruktionen auf Basis von Glasgewirketrägern, die mit wasseraktivierbaren Reaktivharzen aus Polyurethan beschichtet sind. Derartig dicke thermoplastische Materialien sind aber aufgrund der hohen Biegesteifigkeit, die diese im nichtaktivierten Zustand besitzen, im Vergleich zu weicheren wasseraktivierbaren PU-Systemen im nichtaktivierten Zustand, schlechter handhabbar und können daher nicht als Großrollen gewickelt in einem Ausgabebehälter bereitgestellt werden. Sie müssen dann in Plattenform bereitgestellt werden. Die Plattenmaterialien müssen dann herstellerseitig in zahlreichen Abmessungen für die jeweilige Anwendung zugeschnitten werden und sind so im Handel erhältlich. Nachteilig ist hierbei, dass eine Vielzahl von verschiedenen Größen in Krankenhäusern oder Praxen vorgehalten werden muss. Darüber hinaus kann bei der Verwendung von Platten vorgegebener Dicke die Steifigkeit und Stabilität nicht variiert werden und ist nicht nach Bedarf modifizier- und anpassbar. Auch entsteht beim Anpassen an verhältnismäßig kleinen Gliedmaßen und Gelenken sowie anderen Körperteilen viel Abfall, während die Platten bei überlangen Körperteilen vielleicht schon wieder zu klein sind.

Alternativ kann eine entsprechende Mindestdicke von 3 bis 4 mm auch durch Übereinanderstapeln von dünnen Thermoplastlagen erreicht werden, die dann wesentlich flexibler bezüglich der Einzellage sind. Derartige Lagenstapel können auch in vorgefertigten Dimensionen je nach zu versorgendem Körperteil in Schachteln oder Kassetten verpackt konfektioniert sein, wobei je nach Anwendung mehrere Lagen entnommen und gemeinsam aktiviert werden können. Hierbei verbleibt das Problem des Abfallanfalls und der Wirtschaftlichkeit sowie des vorrätig zu haltenden Sortiments, um alle möglichen Anwendungen abdecken zu können. Darüber hinaus sind für entsprechende Stapel Stanz- oder Schneidschritte notwendig und das Handling mehrerer Lagen ist umständlich und unbequem.

Ausgehend von diesem Stand der Technik stellt sich nun der Erfindung die Aufgabe, ein System zur Herstellung eines orthopädischen Splints aus einem Castmaterial mit wenigstens einem als Flachmaterialbahn vorliegenden thermoplastischen Castmaterial bereitzustellen, bei dem das Castmaterial in Rollenform in eine Ausgabebox konfektioniert sein kann und die Bereitstellung eines Splintrohlings in der erforderlichen Mindestdicke für den Anwender einfach herstellbar ist.

Gelöst wird diese Aufgabe durch ein System zur Herstellung eines orthopädischen Splints aus einem Castmaterial, wobei das System wenigstens ein als Flachmaterialbahn vorliegendes thermoplastisches Castmaterial sowie eine Schablone mit einem flächig ausgeführten Schablonenkörper umfasst, wobei der Schablonenkörper in einer vorgegebenen Richtung zur Erstellung eines Splintrohlings mit dem Castmaterial umwickelt und der Splintrohling zur Erzielung einer Verformbarkeit aktivierbar ist, insbesondere, sofern erforderlich, nach Ablängen des Castmaterials auf eine gewünschte Länge.

Darüber hinaus wird die Erfindung durch ein Verfahren zum Herstellen eines orthopädischen Splints mit folgenden Schritten gelöst:
a) Umwickeln eines Schablonenkörpers einer Schablone in vorgegebener Richtung mit einem als Flachmaterialbahn vorliegenden thermoplastischen Castmaterial mit einer vorgegebenen Anzahl von Wicklungen;
b) Fixieren eines außen liegenden freien Endes des Castmaterials zur Bildung eines Splintrohlings;
c) Aktivieren des Splintrohlings zur Erzielung einer plastischen Verformbarkeit und
d) Applizieren sowie Anformen an eine Gliedmaße oder einen Körper bzw. ein Gelenk.

Durch die vorliegende Erfindung wird die Herstellbarkeit eines entsprechenden Splintes erheblich vereinfacht. So dient die Schablone, die aus einem flächigen Schablonenkörper gebildet sein kann, als Wickelkern, wobei das Castmaterial im ersten Schritt vorzugsweise aus einer Ausgabebox herausgezogen werden kann, wobei das Castmaterial als quasi endlos Flachbahnmaterial vorliegt. Entsprechendes Castmaterial, das in Rollenform definierter Geometrie vorliegt, besitzt einen Rollendurchmesser von ca. 20 bis 50 cm und eine Breite von 8 bis 12 cm. Die Breite ist dabei in handelsüblicher Breite vorgebbar.

Im Gegensatz zu Splint-Materialien auf Basis reaktiver, wasserhärtender Polyurethanharze, bei denen zur Vermeidung des vorzeitigen Aushärtens durch Luftfeuchtigkeit eine hermetisch dichte (wasserdampfdichte) Verpackungsfolie als Primärverpackungshülle um das Castmaterial erforderlich ist, kann das nicht-aktivierte Castmaterial bei thermoplastischen Castmaterialrollen ohne wasserdampfdichte Hülle in einer Standard-Pappkarton-Schachtel gelagert werden. Die Lagerbeständigkeit ist deutlich verbessert, da das thermoplastische Material nicht vorzeitig aushärtet und bis zur eigentlichen Aktivierung sowohl bei Kälte (unter 0°C) als auch Wärme (bis 55°C) mehrere Jahre gelagert werden kann.

Der den Splint Herstellende sucht dann zunächst eine Schablone aus, die hinsichtlich ihrer Länge und/oder Breite zum einen der Breite des Castmaterials und zum anderen der Länge der zu versorgenden Gliedmaße bzw. des Körperteils oder Gelenks entspricht, so dass ein späterer Splint eine ausreichende Längserstreckung-aufweist. Die Schablone kann dabei, sofern erforderlich, auch hinsichtlich der Form an den zu versorgenden Körperteil angepasst sein. Das Castmaterial wird dann in der benötigten Lagenzahl um die Schablone, die als Wickelkern dient, herumgewickelt, wobei eine vollständige Umdrehung um die Schablone zwei Lagen in zu fertigenden Splint entspricht.

Je nach Länge der Flachmaterialbahn kann das Castmaterial vor dem Fixieren eines außenliegenden freien Endes abgelängt werden.

Das Aufwickeln kann dabei vergleichsweise lose erfolgen, sofern das Material solche Rückzugskräfte besitzt, dass es sich beim nachfolgenden Aktivieren an die Schablone anlegt und anpasst.

Besonders bevorzugt ist hierbei, dass die Lagen so aufgewickelt werden, dass die Längskanten des Castmaterials bündig übereinander aufliegen. Das Flachbahnmaterial wird dann nach Umwicklung der Schablone mit der gewünschten Anzahl von Umdrehungen und so der gewünschten Anzahl von Lagen für den späteren Splint vorzugsweise so abgelängt, dass auf beiden Seiten der Schablone die gleiche Anzahl von Lagen vorgesehen ist und das Flachbahnmaterial in Längsrichtung mit einer Querkante der Schablone abschließt. Auf diese Weise wird sichergestellt, dass in allen Bereichen über die Längserstreckung des Splints ein gleich starker Verband erzielt werden kann und so eine gleichbleibende Stabilität gegeben ist.

Sofern dies erwünscht ist, kann jedoch auch zur Veränderung der Stabilität vorgesehen sein, Anfang und/oder Ende des Castmaterials, also der Flachmaterialbahn, nicht mit einer Querkante der Schablone beginnen zu lassen, sondern in Längsrichtung in einer gewünschten vorgegebenen Position der Schablone mit dem Umwickeln zu beginnen, so dass der Splint über seine Länge verschiedene Stabilitäten aufweist, da nicht überall die gleiche Lagenanzahl vorliegt.

Es kann dann bevorzugt vorgesehen sein, dass das freie Ende des Castmaterials, das sich nicht innerhalb der Umwicklungen durch das Castmaterial befindet, beispielsweise mit einer Klammer an der Schablone festgelegt wird. Der Rest des Castmaterials kann z. B. im Vorratsbehälter, also insbesondere dem Ausgabebehälter, verbleiben.

Der so gebildete Splintrohling wird dann zusammen mit der Schablone oder nach Trennung von der Schablone aktiviert, um diesen verformbar zu machen. Bei einem thermoplastischen Castmaterial wie dem vorliegenden erfolgt hierbei die Aktivierung durch Erwärmung, beispielsweise im Wasserbad oder mittels eines Heißluftofens, wodurch das thermoplastische Castmaterial in einen plastischen und damit formbaren Zustand überführt wird.

Nach einem bevorzugten Ausführungsbeispiel kann nun vorgesehen sein, dass bei dem aktivierten Splintrohling, bevor dieser auf eine Gliedmaße bzw. einen Körper oder ein Gelenk appliziert wird und dort angeformt wird, die Schablone zuvor entnommen wird. Alternativ kann jedoch auch vorgesehen sein, wobei dies vom Schablonenmaterial abhängig ist, dass die Schablone gemeinsam mit dem Splintrohling appliziert und an einem Patienten angeformt wird. Als Patient können dabei sowohl Menschen als auch Tiere dienen. Nach der Applikation und der Anformung erfolgt dann eine Aushärtung, wobei hierbei vorgesehen sein kann, dass während des Aushärtprozesses der Splint am Patienten mittels geeigneter Fixiermittel, beispielsweise einer Bandage, befestigt ist und nach Abkühlen und damit Erstarren zu einer festen Schiene mit gutem Lagenverbund eine gewünschte Stützwirkung bereitstellt.

Dabei kann des Weiteren vorgesehen sein, dass mit diesem System sowie dem Verfahren mehrlagige thermoplastische Splints in kurzer Zeit auf den jeweiligen Patienten hin individuell in der jeweilig benötigten bzw. gewünschten Stützwirkung und damit Stabilität und Steifigkeit angefertigt werden können, wobei auch über die Wahl der entsprechenden Schablone die Form und Länge des Splints frei wählbar ist. Die Stabilität kann dabei über die Anzahl der Lagen und damit über die Anzahl der Umwicklungen der Schablone eingestellt werden. Auf diese Weise entfällt ein mühsames Schneiden und Stapeln von einzelnen Lagen übereinander, wie es bei der bisherigen Longuettentechnik notwendig ist. Darüber hinaus ist es auch nicht notwendig, eine Vielzahl von vorgefertigten Longuettengrößen bereitzuhalten, die Stauraum belegen. Auf diese Weise kann mittels der in dem System vorgesehenen Schablone die Effektivität für Anwender und Patient erhöht werden und so neben einer variablen Steifigkeit die Sortimentsgestaltung wirtschaftlicher gestaltet werden.

Das Entfernen der Schablone erfolgt dabei durch einfaches Herunterstreifen des aktivierten Splintrohlings nach Entfernen, sofern vorgesehen, der Fixierung des freien Endes des Castmaterials. Dabei kann eine Klammer oder Entsprechendes zur Fixierung des freien Endes im System integriert sein und kann vorzugsweise zusammen mit der Schablone gelagert werden. Insbesondere kann ein entsprechendes Fixiermittel, wie eine Klammer, während der Lagerung an der Schablone festgelegt sein, so dass Schablone und Klammer stets zusammen zur Hand sind.

Wenn im Zusammenhang mit der vorliegenden Erfindung von einer Schablone die Rede ist, ist hiermit mindestens eine Schablone gemeint. Insbesondere kann ein System auch zwei, drei, vier oder eine Mehrzahl an Schablonen umfassen, wobei vorzugsweise jede Schablone in mindestens einer Dimensionierung von allen -anderen Schablonen verschieden ist. Insbesondere kann ein erfindungsgemäßes Set ein Set von Schablonen für eine Körpergliedmaße, z. B. einen Unterarm oder einen Unterschenkel, umfassen, wobei jede Schablone des Sets in mindestens einer Dimension verschieden von den übrigen Schablonen des Sets ist.

Des Weiteren kann vorgesehen sein, dass besonders bevorzugt die Schablone längen- und/oder breitenverstellbar ist. Die Längenverstellbarkeit, aber auch Breitenverstellbarkeit, kann entweder in Form einer Teleskopierbarkeit der Schiene oder gemäß einem Auszugsprinzip, wie es bei einer Schieblehre vorgesehen ist, erzielt werden. Auf diese Weise kann mit einer einzigen Schablone gearbeitet werden und es müssen nicht verschiedene Schablonen für verschiedene Anwendungen und Patienten bereitgestellt werden. Dabei kann eine individuelle und gegebenenfalls sogar stufenlose Einstellbarkeit vorgesehen sein, so dass an die Gliedmaßenlänge eines jeden Patienten individuell eine Anpassung erfolgen kann. Aber auch eine Verstellung in diskreten Stufen ist möglich. Eine Breitenverstellung besitzt insbesondere dann Vorteile, wenn Castmaterialien verschiedener Breiten, wie beispielsweise die handelsüblichen Breiten von 8, 10, 12,5 und 20 cm, verarbeitet werden sollen. Allerdings kann auch mit Schablonen gearbeitet werden, die breiter oder schmaler als die Breite der Flachmaterialbahn sind.

Auf diese Weise kann auch vermieden werden, dass für den Anwendungsfall die falsche, weil zu lange, Schablone zur Hand ist, was entweder dazu führt, dass gegebenenfalls die Schablone nicht verwendet oder beispielsweise doch gekürzt wird, was dann für spätere Anwendungen zu Nachteilen führt.

Sofern die Schablone längenverstellbar vorgesehen ist, kann der Anwender hiermit z. B. das genaue Unterarmmaß eines Patienten abgreifen und die Schablone als Wickelkern zur Anwendung des Splintrohlings verwenden. Damit kann in kurzer Zeit mit wenigen Drehbewegungen beispielsweise eine sechslagige Castschiene mit Individualmaß angefertigt werden. Es ist anschließend ein einziger Schneidevorgang notwendig, wenn das Castmaterial nach dem Wickeln von dem quasi Endlosmaterial im Dispenser getrennt wird.

Die Schablone kann dabei aus einem Holz, einem Kork, aber auch einem Metall- oder Keramikmaterial sowie aus einem Glasmaterial, einem Kunststoff oder einem Verbundwerkstoff bestehen. Darüber hinaus kann die Schablone auch aus verschiedenen Materialien wie Stangen, Drähten, Bügeln, etc. montiert sein, wobei mehrere Elemente miteinander kombiniert werden können. Je nach Material und abhängig davon, ob die Schablone mitappliziert werden soll und im fertigen Splint verbleibt, kann das Schablonenmaterial so gewählt werden, dass es zur weiteren Stabilisierung des Splintes dient. In diesem Fall ist es erforderlich, mehrere Schablonen gemeinsam mit dem Castmaterial bereitzustellen, da diese als Wegwerfmaterial zu betrachten sind. Sofern die Schablone wiederverwendbar ist, ist die Dauerhaftigkeit des Schablonenmaterials von Relevanz.

Die Form der Schablone kann dabei an die vorgesehene Verwendung des Splints angepasst sein und insbesondere können rechteckige, aber auch trapezförmige oder abgerundete sowie gezackte Schablonen bereitgestellt werden.

Dabei kann ebenfalls vorgesehen sein, dass die Schablone vollflächig, also als Plattenmaterial, bereitgestellt wird. Es sind jedoch auch perforierte oder gelochte sowie gitterartige Ausgestaltungen der Schablone möglich.

Darüber hinaus kann vorgesehen sein, dass die Schablone einen oder zwei-seitliche Anschläge aufweist. Zwei seitliche Anschläge können besonders bevorzugt zusammen mit einer Breitenverstellbarkeit eingesetzt werden, da in diesem Fall auch für verschieden breit vorliegende Rollenmaterialien des Castmaterials nur eine Schablone benötigt wird. Die Vorsehung mindestens eines seitlichen Anschlags bietet den Vorteil, dass ein bündiges Übereinanderwickeln der Längskanten des Flachbahnmaterials besonders einfach realisiert werden kann.

Nach einer weiteren Ausführungsform kann vorgesehen sein, dass die Schablone durch den Ausgabebehälter gebildet wird. D. h., in diesem Fall wird das Castmaterial um den Ausgabebehälter herumgewickelt, abgeschnitten und vom Karton entfernt.

Weiterhin kann auch vorgesehen sein, dass die Schablone mit dem Ausgabebehälter, der vorzugsweise aus einem Kartonmaterial besteht, fest oder lösbar verbunden ist. Vorzugsweise werden die Schablonen jedoch als separate Elemente geliefert, die gemeinsam oder insbesondere in einem Ausgabebehälter enthalten sind und so zusammen mit diesem an die Anwender ausgeliefert werden.

Das Umwickeln der Schablone kann manuell, aber auch mittels einer Halte- oder Drehvorrichtung, die insbesondere eine Handkurbel beinhalten kann, erfolgen.

Darüber hinaus kann vorgesehen sein, dass vor Anlegen an einen Patienten der Splintrohling quer zu seiner Längserstreckung, insbesondere entlang der Stelle, die durch die beiden freien Enden des Castmaterials begrenzt ist, aufgeschnitten wird, und so zu einem flachen Element aufgeklappt wird. So kann beispielsweise aus einem sechslagigen ringförmigen Körper durch einen einfachen Schnitt ein dreilagiger Splintrohling in doppelter Länge bereitgestellt werden. Etwas Entsprechendes ist insbesondere im Veterinärbereich bei der Anwendung von langen Schienen an Tierbeinen, wie beispielsweise Pferdebeinen, interessant, da durch die Wicklung um eine nur halb so lange Schablone, die dadurch jedoch eine größere Stabilität und bessere Handhabbarkeit besitzt, und dann Öffnen des Wickelkörpers zu einem halb so dicken, aber doppelt so langen Flachmaterial die Erzielung derartiger Splintrohlinge deutlich erleichtert werden kann.

Schließlich umfasst die Erfindung ein Verfahren zum Herstellen eines orthopädischen Splints mit den vorstehend genannten Schritten, wobei ein derartiges Verfahren den Vorteil besitzt, besonders einfach und schnell einen Splint bereitstellen zu können.

Ferner kann erfindungsgemäß vorgesehen sein, dass vor Schritt a), nämlich dem Umwickeln des Schablonenkörpers, eine Anpassung der Schablonenlänge an einen Patienten und dort an einen zu versorgenden Körperteil erfolgt. Auch kann eine Auswahl der geeigneten Schablone vorgeschaltet sein.

Ebenfalls kann vorgesehen sein, vor dem Applizieren sowie Anformen an eine Gliedmaße die Schablone zu entfernen.

Eine Aktivierung des Castmaterials kann hierbei im Wasserbad oder auch in einem Ofen erfolgen.

Beim Aktivieren im Wasserbad wird dabei vorzugsweise der gesamte Splintrohling inklusive Schablone ins Wasserbad eingelegt. Eine Aktivierung auch bei sechslagiger Bewicklung der Schablone in einem Wasserbad mit einer Temperatur von 70°C bis 95°C ist in ca. 30 bis 60 sec abgeschlossen. Durch die Rückzugskraft des thermoplastischen Castmaterials passt sich dieses der Schablonenform optimal an, kann aber auch leicht, da nun in der Wärme plastisch geworden, verformt werden und so von der Schablone abgezogen werden. Anschließend werden dann die Lagen miteinander verpresst, um eine bessere Verbindung des Lagenverbundes zu erreichen und der Splintrohling wird dann an einen zu behandelnden Körperteil anmodelliert.

Bei dem für das System sowie das Verfahren verwendeten thermoplastischen Material kann es sich insbesondere um ein solches handeln, bei dem auf eine erste Textilbahn ein thermoplastischer Kunststoff aufgebracht ist, wobei auf den thermoplastischen Kunststoff vorzugsweise eine zweite Textilbahn aufgelegt werden kann. Der Vorteil, der durch die zweite Textilbahn realisiert wird, liegt in der besseren Abrollbarkeit, sogar nach kräftigem Ausdrücken des Restwassers nach Erwärmen in einem Wasserbad, wobei die zweite Textilbahn die Lagenhaftung im fertigen Splintmaterial nicht nachteilig beeinflusst. Darüber hinaus trägt die zweite Textilbahn zur Stabilität und zur Verbesserung der Luftdurchlässigkeit bei.

Dabei kann vorzugsweise vorgesehen sein, dass die erste und/oder die zweite Textilbahn dehnbar gestaltet sind. Der thermoplastische Kunststoff weist dabei bevorzugt einen Schmelzpunkt von 55°C bis 90°C auf, wobei er bei Temperaturen von 50°C oder darunter starr oder restflexibel, aber nicht wesentlich erweicht ist und im plastischen Zustand selbstklebend ist.

Für die Textilbahnen können hierbei Vliesbahnen, aber auch Gelege oder Gewirke, Gewebe oder Gestricke eingesetzt werden. Vorzugsweise kann ein gewirktes Band mit offenporiger Struktur vorgesehen sein.

Der Begriff "Dehnbar" umfasst hierbei sowohl unelastische als auch elastische Dehnbarkeit.

Die Textilbahnen können hierbei aus synthetischen, regenerierten und natürlichen Fasern sowie Mischungen hieraus bestehen, wobei ebenfalls Mischungen aus elastischen und unelastischen Fasern bzw. Fäden eingesetzt werden können. Besonders bevorzugt sind dabei Textilbahnen aus synthetischen Fäden bzw. Fasern aus einem oder mehreren synthetischen Materialien, z. B. Polyester oder Polyamid, als nichtelastische Garne, und Polyurethanfäden bzw. -fasern als elastische Garne.

Die Dehnbarkeit kann dabei sowohl in Längs- als auch in Querrichtung bestehen und beträgt vorzugsweise für die Längsdehnbarkeit 30 bis 200%, weiter bevorzugt 60 bis 110% und besonders bevorzugt 85 bis 100%. Eine Querdehnbarkeit kann vorzugsweise zwischen 10 und 120%, weiter bevorzugt 30 bis 100% und besonders bevorzugt 40 bis 90% betragen. Die Dehnbarkeit wird hierbei nach dem in DIN 61632 beschriebenen Verfahren ermittelt.

Derartige Castmaterialien können zumeist weiß sein, wobei hier insbesondere die Farbe der Textilbahn maßgeblich ist. Es besteht jedoch auch die Möglichkeit, gefärbte Textilbahnen einzusetzen.

Als thermoplastischer Kunststoff kann ein hydrolysestabiler lagerbeständiger Hotmeltklebstoff, der bei Temperaturen von 55°C bis 90°C, vorzugsweise 60°C bis 80°C und besonders bevorzugt 60°C bis 70°C schmilzt und auch nach Abkühlen unter den Schmelzpunkt für einige Zeit plastisch bleibt, eingesetzt werden. Damit der thermoplastische Kunststoff in einem thermoplastischen Verbandmaterial unter den normalen Anwendungsbedingungen einsetzbar ist, muss er eine Temperaturbeständigkeit bis 50°C besitzen, vorzugsweise bis 55°C. D. h., es darf bei diesen Temperaturen nicht zu einer wesentlichen Erweichung oder gar zu einer Zersetzung des Kunststoffes kommen.

Die Aushärtezeit nach dem Erwärmen auf oder über den Schmelzpunkt hängt von der erreichten Temperatur und der Abkühlgeschwindigkeit ab und beträgt im Allgemeinen zwischen 1 und 15 min, vorzugsweise 2 bis 10 min, besonders bevorzugt 3 bis 8 min.

Geeignete thermoplastische Kunststoffe mit den oben genannten Eigenschaften sind beispielsweise Polyester, Polyurethan, Polyvinylacetat oder auch weitere Kunststoffe, wie beispielsweise linear gesättigte Polyesterverbindungen, wie das kommerziell erhältliche Polycaprolakton CAPA R 640 (Hersteller: SOLVAY-INTEROX, Warrington, GB), ein Polycaprolakton in Granulatform mit einem Schmelzpunkt von 57°C). Ein Beispiel für ein geeignetes thermoplastisches Polyurethan ist das Handelsprodukt UNEX 4103 (Hersteller: DAKOTA COATINGS, Neerhonderd, Belgien). Es können jedoch auch Mischungen verschiedener thermoplastischer Kunststoffe verwendet werden, darüber hinaus können Hilfs- und Zusatzstoffe zugegeben werden. Auch einsetzbar sind thermoplastische Kunststoffe, die im erstarrten Zustand eine gewisse Restflexibilität besitzen, anstatt vollkommen zu erstarren. Beispiele hierfür sind Ethylen-Acrylsäureester-Copolymere, Ethyl-Vinylacetat-Copolymere und Polyurethane. Durch den Einsatz solcher Verbandmaterialien wird keine vollständige Immobilisierung des mit dem hergestellten Verband versehenen Körperteils bewirkt, sondern lediglich eine semirigide Stabilisierung, die eine funktionelle Belastung der betroffenen Körperstellen ermöglicht.

Die Herstellung eines entsprechenden thermoplastischen Castmaterials kann gemäß der DE 199 07 043 B4 erfolgen.

Die weiteren Vorteile und Merkmale der Erfindung ergeben sich aus den übrigen Anmeldungsunterlagen. Ein Ausführungsbeispiel der Erfindung ist nun in der Zeichnung erläutert.

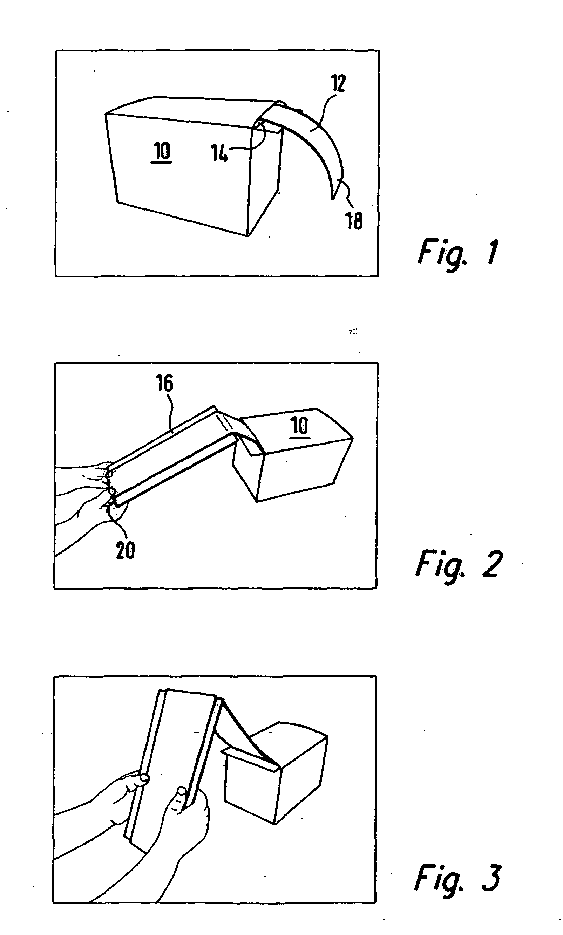

Dabei zeigen die Figuren 1 bis 10 den Verfahrensablauf zur Herstellung eines orthopädischen Splints mit einem erfindungsgemäßen System sowie nach einem erfindungsgemäßen Verfahren.

Figur 1 zeigt einen Ausgabebehälter 10, in dem ein Castmaterial auf Basis eines thermoplastischen Kunststoffes 12 als quasi endlos Rollenmaterial bestehend aus einer Flachmaterialbahn beinhaltet ist. Die Box wird zur Benutzung geöffnet und durch eine Ausgabeöffnung 14 wird ein freies Ende 18 des Castmaterials herausgezogen, so dass dieses durch einen Anwender ergriffen werden kann.

Zur Herstellung eines orthopädischen oder medizinischen Splints wird nun zunächst an der zu behandelnden Person oder dem zu behandelnden Tier Maß genommen und eine Wickelschablone 16 der gewünschten Breite abgestimmt auf die Breite des Castmaterials 12 sowie abgestimmt auf die Länge der mit dem Splint zu versorgenden Gliedmaße gewählt. Das freie Ende 18 des thermoplastischen Castmaterials wird auf eine Querkante 20 der Schablone 16 aufgelegt und dort festgehalten. Das Castmaterial wird dann unter Herausziehen aus dem Behälter 10 um die Schablone 16 herumgewickelt. Jeweils eine Wicklung um 360° um die Schablone 16 ergibt hierbei zwei Lagen für einen späteren Splint. Dabei zeigt Figur 3 den Wickelvorgang, wobei Figur 2 den Beginn der Umwicklung darstellt. Durch fortgesetztes Drehen der Schablone 16 wird die gewünschte Lagenanzahl erreicht, wobei vorgesehen ist, dass bevorzugt die einzelnen Lagen hinsichtlich ihrer Längskanten bündig aufeinander liegen.

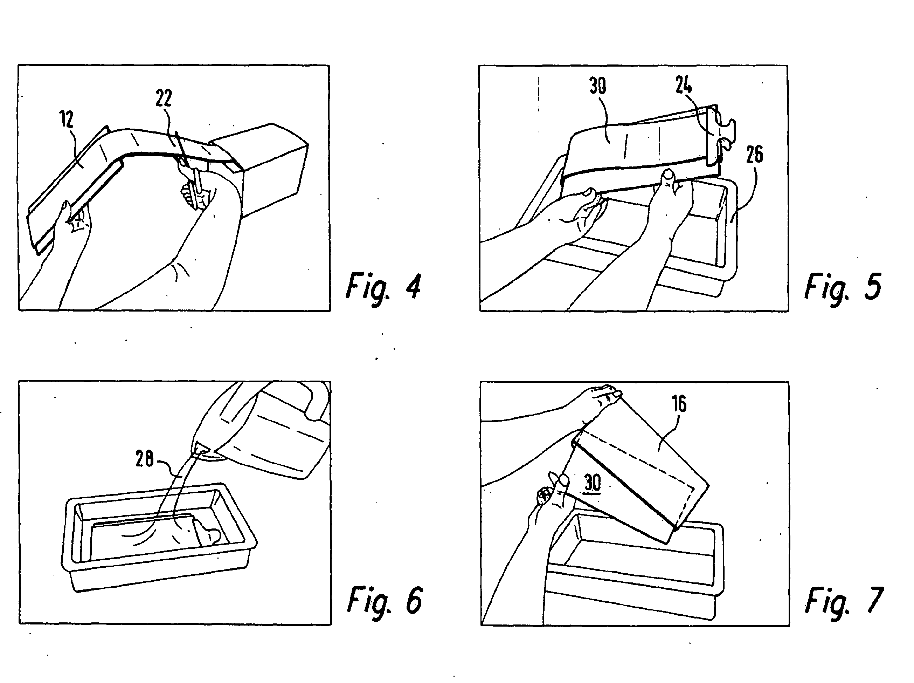

Nach beispielsweise vier Drehungen der Schablone 16 ist ein achtlagiger Splintrohling bereitgestellt, wie ihn Figur 4 zeigt.

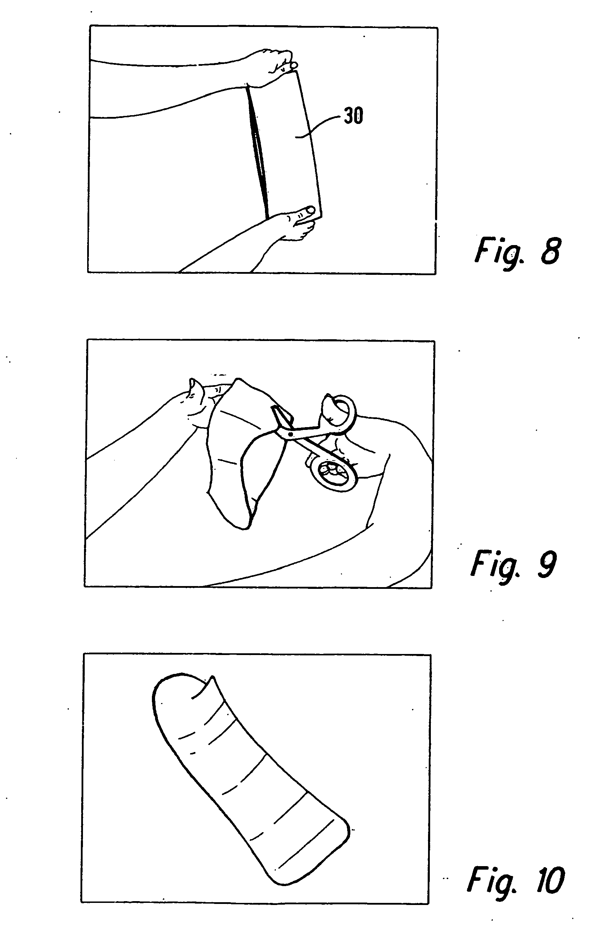

Mittels eines einfachen Schnitts kann dann das Castmaterial 12 abgelängt werden, wobei das Ablängen vorzugsweise so erfolgt, dass das freie Ende 22 des Castmaterial, das durch Ablängung gebildet ist, wiederum mit der Querkante 20 oder der anderen Querkante der Schablone-16 bündig abschließt. Das freie Ende 22 wird dann mittels eines Fixiermittels, hier einer Klammer 24, an der Schablone 16 festgeklemmt (Figur 5). Alternativ kann jedoch das freie Ende 22, aber auch der Anfang des Castmaterials nicht bündig mit einem Querrand der. Schablone 16 abschließen. Der so entstandene Splintrohling wird dann in eine Aktivierungseinrichtung, beispielsweise ein Wasserbad, gegeben, wobei das Wasserbad hier durch ein Behältnis 26 angedeutet ist, in das entsprechend erwärmtes Wasser 28 eingefüllt wird (Figur 6). Je nach Lagenzahl und bei vollständigem Eintauchen des Splintrohlings, der in seiner Gesamtheit mit dem Bezugszeichen 30 bezeichnet ist, ist nach 30 bis 60 sec Verweilzeit eine Aktivierung durch Wärme des thermoplastischen Kunststoffs erreicht. Der Splintrohling 30 kann dann aus dem Wasserbad entnommen werden und die Schablone 16 wird aus dem Splintrohling 30 herausgezogen und dadurch aus diesem entfernt, wie dies in Figur 7 dargestellt ist. Figur 8 zeigt nun den fertigen Splintrohling 30, der nun erwärmt und gleichzeitig plastisch verformbar ist. Gemäß Figur 8 wird der Splintrohling 30 nun glattgezogen, ausgepresst und glattgestrichen, um überschüssiges Wasser zu entfernen und einen guten Lagenverbund zu erreichen.

Figur 9 stellt nun eine optionale Gestaltung dar, bei der der Wickelkörper, den der Splintrohling 30 bildet, an einer Stelle in Querrichtung durchtrennt wird, so dass ein Splintrohling mit halber Lagenzahl, aber doppelter Länge erhalten wird. Etwas Entsprechendes kann insbesondere für veterinärmedizinische Anwendungen vorteilhaft sein.

Figur 10 zeigt nun den fertigen Splint, wobei der Splintrohling gemäß Figur 8 auf eine zu behandelnde und zu stabilisierende Gliedmaße appliziert und hier angeformt und beispielsweise mittels einer Bandage an der gewünschten Stelle und in der gewünschten Haltung fixiert wurde, bis der Splintrohling zum fertigen Splint 40 ausgehärtet ist. Danach kann der Splint 40 in gewünschter Weise an einer Gliedmaße oder einem Gelenk eines Patienten angebracht werden und dient dann zur Stabilisierung derselben.

## Patentansprüche

1. System zur Herstellung eines orthopädischen Splints (40) aus einem Castmaterial (12), wobei das System wenigstens ein als Flachmaterialbahn vorliegendes thermoplastisches Castmaterial (12) sowie eine Schablone (16) mit einem flächig ausgeführten Schablonenkörper umfasst, wobei der Schablonenkörper in einer vorgegebenen Richtung zur Erstellung eines Splintrohlings (30) mit dem Castmaterial (12) umwickelbar ist und der Splintrohling (30) zur Erzielung einer Verformbarkeit aktivierbar ist, insbesondere nach Ablängen des Castmaterials (12).

2. System nach Anspruch 1, bei dem der aktivierte Splintrohling (30) auf eine Gliedmaße oder einen Körper applizier- und anformbar und in dieser Form zu einem Splint (40) aushärtbar ist.

3. System nach Anspruch 1 oder 2, bei dem die Schablone (16) vor oder nach dem Aktivieren entnehmbar ist.

4. System nach einem der Ansprüche 1 bis 3, bei dem nach dem Ablängen das freie Ende (22) des Castmaterials (12) fixierbar ist.

5. System nach einem der vorangehenden Ansprüche, bei dem der Splintrohling (30) zusammen mit der darin vorliegenden Schablone (16) applizier- und anformbar ist.

6. System nach einem der vorangehenden Ansprüche, bei dem das Castmaterial (12) als quasi Endlosmaterial, vorzugsweise in Rollenform konfektioniert ist.

7. System nach einem der vorangehenden Ansprüche, bei dem die Schablone (16) längenverstellbar und/oder breitenverstellbar ist.

8. System nach einem der vorangehenden Ansprüche, bei dem das Castmaterial (12) in einem Ausgabebehälter (10) vorliegt.

9. System nach Anspruch 8, bei dem die Schablone (16) durch den Ausgabebehälter (10) gebildet ist oder mit dem Ausgabebehälter (10) verbunden ist, insbesondere unmittelbar hiermit verbunden ist.

10. System nach einem der vorangehenden Ansprüche, bei dem die Schablone (16) mit einer Halte- und/oder Drehvorrichtung verbindbar ist.

11. System nach einem der vorangehenden Ansprüche, bei dem die Schablone (16) vollflächig oder mit Aussparungen ausgebildet ist.

12. System nach einem der vorangehenden Ansprüche, bei dem die Schablone-(16) einen oder zwei seitliche Anschläge aufweist zum Ermöglichen eines randgenauen Wickelns des Castmaterials (12).

13. System nach einem der vorangehenden Ansprüche, bei dem das System mehrere Schablonen (16), die gleich oder voneinander verschieden sind und vorzugsweise ein Set von Schablonen (16) umfasst, wobei jede Schablone (16) in mindestens einer Dimension verschieden von den übrigen Schablonen (16) des Sets ist.

14. Verfahren zum Herstellen eines orthopädischen Splints mit folgenden Schritten:
a) Umwickeln eines Schablonenkörpers einer Schablone in vorgegebener Richtung mit einem als Flachmaterialbahn vorliegenden thermoplastischen Castmaterial mit einer vorgegebenen Anzahl von Wicklungen,
b) Fixieren eines freien außenliegenden Endes des Castmaterials zur Bildung eines Splintrohlings,
c) Aktivieren des Splintrohlings zur Erzielung einer plastischen Verformbarkeit und
d) Applizieren sowie Anformen an eine Gliedmaße oder einen Körper.

15. Verfahren nach Anspruch 14, bei dem vor Schritt a) die Schablone ausgewählt und/oder hinsichtlich ihrer Länge und/oder Breite angepasst wird.

16. Verfahren nach Anspruch 14 und 15, bei dem vor Schritt d) die Schablone entfernt wird.

17. Verfahren nach einem der vorangehenden Ansprüche, bei dem vor dem Fixieren des freien außenliegenden Endes das Castmaterial abgelängt wird.

18. Verfahren nach einem der vorangehenden Ansprüche, bei dem zur Verdoppelung der Länge des Splints und Halbierung der vorgegebenen Lagenzahl vor Schritt c) die Aufwicklung des Castmaterials quer zur Wickelrichtung aufgeschnitten wird.

19. Verfahren nach einem der vorangehenden Ansprüche, bei dem die Aktivierung als Wärmeaktivierung erfolgt.

## Claims

1. A system for producing an orthopedic splint (40) made of a cast material (12), wherein the system at least comprises a cast material (12) available as a flat material layer as well as a template (16) having a flat template body, wherein the cast material (12) may be wound around the template body in a prescribed direction for generating a splint blank (30), and the splint blank (30) may be activated in order to achieve deformability, particularly after cutting the cast material (12) to length.

2. The system according to claim 1, wherein the activated splint blank (30) may be applied and adapted to a limb or a body and be hardened in this shape to form a splint (40).

3. A system according to any one of claims 1 or 2, wherein the template (16) may be removed before or after the activation.

4. A system according to any one of claims 1 to 3, wherein the free end (22) of the cast material (12) may be fixed after having been cut to length.

5. A system according to any one of the preceding claims, wherein the splint blank (30) may be applied and adapted together with the template (16) contained therein.

6. A system according to any one of the preceding claims, wherein the cast material (12) is ready-made as quasi endless material preferably in the form of a roll.

7. A system according to any one of the preceding claims, wherein the template (16) may be adjusted lengthwise and/or widthwise.

8. A system according to any one of the preceding claims, wherein the cast material (12) is available in a dispensing container (10).

9. The system according to claim 8, wherein the template (16) is formed via the dispensing container (10), or is connected to the dispensing container (10), in particular directly connected to it.

10. A system according to any one of the preceding claims, wherein the template (16) may be connected to a holding and/or turning device.

11. A system according to any one of the preceding claims, wherein the template (16) covers the entire area or is configured with cut-outs.

12. A system according to any one of the preceding claims, wherein the template (16) has one or two lateral stops in order to enable the cast material (12) to be wound exactly to the edge.

13. A system according to any one of the preceding claims, wherein the system comprises several templates (16), which are similar or different from one another, and preferably comprises a set of templates (16), wherein each template (16) is at least different in one dimension from the remaining templates (16) of the set.

14. A method for producing an orthopedic splint according to the following steps:
a) winding a thermoplastic cast material, which is available as a flat material layer, around a template body of a template in a prescribed direction with a specified number of turns;
b) fixing an external free end of the cast material to form a splint blank;
c) activation of the splint blank to accomplish plastic deformability, and
d) application as well as adaptation to a limb or body.

15. The method according to claim 14, wherein the template is selected, and/or adapted with regard to its length and/or width prior to step a).

16. A method according to any one of claims 14 and 15, wherein the template (16) is removed prior to step d).

17. A method according to any one of the preceding claims, wherein the cast material is cut to length prior to fixing the external end.

18. A method according to any one of the preceding claims, wherein the winding of the cast material is cut open transversely to the winding direction in order to double the length of the splint and halving the specified number of layers prior to step c).

19. A method according to any one of the preceding claims, wherein the activation is carried out as heat activation.

## Revendications

1. Système pour fabriquer une attelle orthopédique (40) à partir d'une matière de moulage (12), le système comprenant au moins une matière de moulage thermoplastique sous la forme d'une bande de matériau plat (12) et un moule (16) présentant un corps de moule réalisé plat, le corps de moule pouvant être enroulé de la matière de moulage (12) dans une direction déterminée en vue de former une ébauche d'attelle (30) et l'ébauche d'attelle (30) pouvant être activée en vue d'obtenir une déformabilité, en particulier après la découpe de la longueur de la matière de moulage (12).

2. Système selon la revendication 1, dans lequel l'ébauche d'attelle (30) activée peut être appliquée et façonnée sur un membre ou un corps et être durcie dans cette forme pour former une attelle (40).

3. Système selon la revendication 1 ou 2, dans lequel le moule (16) peut être retiré avant ou après l'activation.

4. Système selon l'une des revendications 1 à 3, dans lequel après la découpe de la longueur, l'extrémité libre (22) de la matière de moulage (12) peut être fixée.

5. Système selon l'une des revendications précédentes, dans lequel l'ébauche d'attelle (30) peut être appliquée et façonnée avec le moule à l'intérieur.

6. Système selon l'une des revendications précédentes, dans lequel la matière de moulage (12) est réalisée sous la forme d'un matériau quasi sans fin, de préférence sous forme de rouleau.

7. Système selon l'une des revendications précédentes, dans lequel le moule (16) peut être réglé en longueur et/ou en largeur.

8. Système selon l'une des revendications précédentes, dans lequel la matière de moulage (12) est présentée dans un récipient distributeur (10).

9. Système selon la revendication 8, dans lequel le moule (16) est formé par le récipient distributeur (10) ou bien est relié au récipient distributeur (10), en particulier est directement relié à ce dernier.

10. Système selon l'une des revendications précédentes, dans lequel le moule (16) peut être relié à un dispositif de retenue et/ou de rotation.

11. Système selon l'une des revendications précédentes, dans lequel le moule (16) est réalisé totalement plat ou avec des évidements.

12. Système selon l'une des revendications précédentes, dans lequel le moule (16) présente une ou deux butées latérales en vue de permettre à la matière de coulage (12) de s'enrouler correctement.

13. Système selon l'une des revendications précédentes, dans lequel le système comprend plusieurs moules (16), identiques ou différents les uns des autres, et comprend de préférence une série de moules (16), chaque moule (16) étant différent au moins au niveau d'une dimension par rapport aux autres moules (16) de la série.

14. Procédé pour fabriquer une attelle orthopédique comprenant les étapes consistant à :
a) enrouler un corps de moule dans une direction déterminée d'une matière de moulage thermoplastique présentée sous la forme d'une bande de matériau plat avec un nombre déterminé d'enroulements,
b) fixer l'extrémité restant libre de la matière de moulage en vue de former une ébauche d'attelle,
c) activer l'ébauche d'attelle en vue d'obtenir une déformabilité plastique et
d) appliquer et façonner sur un membre ou un corps.

15. Procédé selon la revendication 14, dans lequel avant de procéder à l'étape a), on choisit le moule et/ou on l'adapte au niveau de sa longueur et/ou de sa largeur.

16. Procédé selon les revendications 14 et 15, dans lequel avant de procéder à l'étape d), on retire le moule.

17. Procédé selon l'une des revendications précédentes, dans lequel avant de fixer l'extrémité libre, on découpe une longueur de la matière de moulage.

18. Procédé selon l'une des revendications précédentes, dans lequel pour doubler la longueur de l'attelle et diviser en deux le nombre de couches, on découpe avant l'étape c) l'enroulement de la matière de moulage transversalement au sens d'enroulement.

19. Procédé selon l'une des revendications précédentes, dans lequel l'activation se fait sous la forme d'une activation thermique.
